(19) Europäisches Patentamt
European Patent Office
Office européen des brevets

(11) Veröffentlichungsnummer: **0 129 615**
**B1**

(12) # EUROPÄISCHE PATENTSCHRIFT

(45) Veröffentlichungstag der Patentschrift: **05.11.86**

(51) Int. Cl.⁴: **C 07 C 50/24**, C 07 C 46/00

(21) Anmeldenummer: **83111907.8**

(22) Anmeldetag: **28.11.83**

(54) **Verfahren zur Herstellung von Bromanthrachinonen.**

(30) Priorität: **23.06.83 CH 3431/83**

(43) Veröffentlichungstag der Anmeldung: **02.01.85 Patentblatt 85/1**

(45) Bekanntmachung des Hinweises auf die Patenterteilung: **05.11.86 Patentblatt 86/45**

(84) Benannte Vertragsstaaten: **CH DE FR GB IT LI**

(56) Entgegenhaltungen:
**DE-A-2 455 587**
**DE-A-2 720 965**
**DE-A-2 749 416**
**DE-B-2 452 014**
**DE-B-2 654 650**

(73) Patentinhaber: **CIBA- GEIGY AG, Klybeckstrasse 141, CH- 4002 Basel (CH)**

(72) Erfinder: **Somlo, Tibor, Dr., Florastrasse 8, CH- 4127 Birsfelden (CH)**
Erfinder: **Regli, Johann, Passwangstrasse 4, CH- 4127 Birsfelden (CH)**

(74) Vertreter: **Zumstein, Fritz jun., Dr., Dr. F. Zumstein sen. Dr. E. Assmann Dr. R. Koenigsberger Dipl.- Ing. F. Klingseisen Dr. F. Zumstein jun. Bräuhausstrasse 4, D-8000 München 2 (DE)**

EP 0 129 615 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein Verfahren zur Herstellung von Bromanthrachinonen hoher Reinheit mit einem Gehalt an Nebenprodukten von $\leq$ 5%.

Bromanthrachinone sind wichtige Zwischenprodukte für die Farbstoffsynthese. Zu deren Herstellung sind in der Vergangenheit zahlreiche Verfahren entwickelt worden, u.a. die sogenannte Denitrohalogenierung, wonach Nitroanthrachinone durch Umsetzen mit Brom, bzw. Chlor, in die entsprechenden Brom/Chlor-Anthrachinone überführt werden (DE-OS 24 52 014 und DE-OS 24 55 587). Kennzeichnend für dieses bekannte Verfahren ist, dass das Nitroanthrachinon im Gemisch mit einem Chlor- bzw. Bromanthrachinon eingesetzt wird, welches als Verdünnungsmittel wirkt. So hergestelltes Chlor- bzw. Bromanthrachinon weist jedoch nur eine Reinheit von weniger als 95% auf, die für eine direkte Verwendung dieser Produkte in der Farbstoffsynthese nicht ausreicht, da bei derartigen Ausgangsprodukten die Reproduzierbarkeit des Farbtons nicht hinreichend gewährleistet ist (siehe DE-OS 25 31 929 und DE-OS 24 58 022). Zudem liegen die Ausbeuten nach diesem Verfahren zum Teil wesentlich unter 90%, was bei solchen Grossprodukten unbefriedigend ist.

Aufgabe der vorliegenden Erfindung war es daher, ein wirtschaftliches Verfahren zu entwickeln, das die Herstellung von Bromanthrachinonen in hoher Ausbeute mit einer Reinheit von über 95% ermöglicht. Nur Produkte, die dieses Reinheitskriterium erfüllen, lassen sich ohne aufwendige Reinigungsoperationen, wie z.B. fraktionierte Vakuumdestillation, direkt zur Herstellung von Farbstoffen verwerden

Es wurde nun gefunden, dass man Bromanthrachinone überraschenderweise in hoher Reinheit (95 bis 99%ig) auf einfache Art und Weise erhält, indem man auf entsprechende Nitroanthrachinone und zwar solche der Formel 1

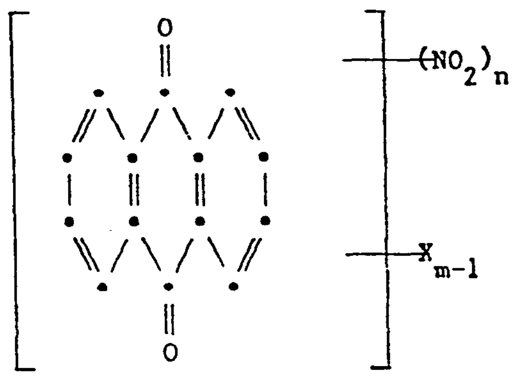

(I),

worin X für die Hydroxy-, Mercapto- oder Carboxygruppe oder Halogen steht; m 1, 2, 3 oder 4 und n 1 oder 2 bedeutet, bei Temperaturen von 200° bis 350° C elementares Brom einwirken lässt.

Die hohe Reinheit der nach diesem Verfahren erhaltenen Produkte ist insofern überraschend, da die Denitrochlorierung bei analoger Verfahrensführung zu Produkten führt, die erhebliche Anteile an schwer entfernbaren Nebenprodukten, wie z.B. Dianthrachinonyle und überchlorierte Anthrachinone, enthalten. Zudem nimmt die Bildung derartiger Nebenprodukte mit steigender Reaktionstemperatur stark zu. Da die Denitrobromierung höhere Temperaturen erfordert als die Denitrochlorierung, musste mit einer vermehrten Bildung von Verunreinigungen gerechnet werden. Widererwarten verläuft die Denitrobromierung jedoch ohne eine nennenswerte Bildung an dimeren, polymeren oder überbromierten Anthrachinonen. Als Nebenreaktion ist praktisch nur die Bildung von Phthalsäureanhydrid zu beobachten, das jedoch nicht weiter stört und sich bei der Aufarbeitung ohne Schwierigkeiten abtrennen lässt.

Um ein leicht rührbares Reaktionsgemisch zu erhalten und die Sublimation des Ausgangsmaterials bzw. des sich bildenden Brom anthrachinons weitgehend zu verhindern, erweist es sich als vorteilhaft, die Denitrobromierung in Gegenwart eines unter Reaktionsbedingungen gegenüber Brom und Stickstoffoxiden inerten organischen Lösungsmittels durchzuführen. Das Lösungsmittel wird zweckmässig in einer Menge von 1 bis 500 Gew.%, bezogen auf Nitroanthrachinon, eingesetzt.

Durch die Verwendung eines Lösungsmittels wird zudem der Schmelzpunkt des zu bromierenden Nitroanthrachinons erniedrigt, was besonders bei Dinitroanthrachinonen von Bedeutung ist, deren Schmelztemperatur bereits in Nähe der Zersetzungstemperatur liegt. Mononitroanthrachinone werden bevorzugt in Gegenwart von 1 bis 100 Gew.% Lösungsmittel und Dinitroanthrachinone vorteilhaft in Anwesenheit von 100 bis 400 Gew.% Lösungsmittel, jeweils bezogen auf Ausgangsmaterial, denitrobromiert.

Als Lösungsmittel gelangen bevorzugt bei Raumtemperatur flüssige Verbindungen mit einem Siedepunkt über 150°C zur Anwendung, wie z.B. halogenierte Nitrobenzole, etwa Nitrofluorbenzol, Nitrochlorbenzol, m-Nitrobenzotrifluorid, Nitrobrombenzol oder Dichlornitrobenzol, ferner Trichlorbenzol, insbesondere jedoch Nitrobenzol. Neben den reinen Lösungsmitteln können auch Gemische zweier oder mehrerer Lösungsmittel verwendet werden.

Durchgeführt wird das erfindungsgemässe Verfahren bei Temperaturen zwischen 200° und 350°C. Unterhalb von 200°C findet praktische keine, bzw. eine für den technischen Maßstab zu langsame Denitrobromierung

statt. Für die obere Temperaturgrenze ist die thermische Beständigkeit der Nitroanthrachinone massgebend. So beginnt sich beispielsweise 1-Nitroanthrachinon bei Temperaturen oberhalb von 370°C langsam zu zersetzen. Bevorzugt wird die Reaktion bei Temperaturen zwischen 230° und 280°C durchgeführt.

Pro Mol zu ersetzender Nitrogruppe verwendet man zweckmässigerweise 0,5 bis 1,5 Mol, insbesondere 0,6 bis 1,0 Mol Brom; natürlich können auch grössere Brommengen zudosiert werden. Das Brom kann der Reaktionsschmelze in flüssiger Form zugesetzt werden, wobei die Verdampfungswärme (30 kJ/Mol) die Reaktionswärme der Denitrobromierung teilweise kompensiert. Es kann aber auch verdampft und dann gasförmig eingeleitet werden.

Der Denitrobromierung gemäss vorliegendem Verfahren werden bevorzugt solche Nitroanthrachinone der Formel 1 unterworfen, die neben einer oder zwei Nitrogruppen keine weiteren Substituenten aufweisen. Dies sind beispielsweise 2-Nitroanthrachinon oder 1,8-Dinitroanthrachinon, insbesondere jedoch 1-Nitroanthrachinon und 1,5-Dinitroanthra-chinon.

Ist die Reaktion beendet und sind die Nitrogruppen vollständig durch Brom ersetzt, was sich leicht durch dünnschichtchromatographische Analyse feststellen lässt, so behandelt man das Reaktionsprodukt gegebenenfalls nach Abkühlen auf eine Temperatur, die oberhalb des Erstarrungspunktes liegt, mit Wasser oder einem organischen Lösungsmittel. Vorteilhaft trägt man das geschmolzene Reaktionsprodukt zur Aufarbeitung in Wasser oder ein organisches Lösungsmittel ein, in dem das Produkt bei 20°C keine oder nur eine geringe Löslichkeit besitzt, und erhitzt gegebenenfalls die so erhaltene Suspension auf 100°C bzw. bis zur Siedetemperatur des jeweils verwendeten Lösungsmittels. Bei dieser Form der Aufarbeitung gehen Nebenprodukte in Lösung und das gewünschte Bromanthrachinon wird in reiner Form als ein leichtfiltrierbares Granulat erhalten. Neben Wasser sind beispielsweise die folgenden organischen Lösungsmittel geeignet: Alkohole, wie Methanol, Isopropanol oder auch höhere Alkohole; Aromaten, wie Toluol, Xylol oder Nitrobenzol; Aliphaten, wie z.B. Isoparaffingemische mit einem Siedebereich zwischen 150° und 210°C, wie die von Esso unter dem Handelsnamen Isopar® vertriebenen Produkte; ferner Ketone, wie Methyläthylketon oder Methylisobutylketon.

Wird die Schmelze in Wasser oder einen niedrig siedenden Alkohol eingetragen, so erhält man Bromanthrachinone mit einem Gehalt von 96 bis 98%; bei Verwendung von Lösungsmittel wie Xylol, Chlorbenzol, Dichlorbenzol oder Nitrobenzol hingegen Produkte mit einem Gehalt bis zu über 99%. Selbstverständlich kann die heisse Reaktionsschmelze auch in einem geeigneten Lösungsmittel, beispielsweise Chlorbenzol oder Dichlorbenzol, gelöst werden, aus dem das Bromanthrachinon beim Abkühlen in reiner Form auskristallisiert. Führt man ferner die Denitrobromierung in Gegenwart grösserer Mengen an Lösungsmittel durch, verwendet man z.B. gleich viel Lösungsmittel wie Nitroanthrachinon, so lässt man zweckmässiger Weise das heisse Reaktionsgemisch langsam auf Raumtemperatur bzw. 0°C abkühlen, wobei das Produkt mit einer Reinheit von 98-99% in kristalliner Form ausfällt.

Das vorliegende Verfahren kann diskontinuierlich oder auch kontinuierlich durchgeführt werden. Chargenweise arbeitet man z.B. folgendermassen:

In einem mit Heizmantel versehenen Reaktor, ausgerüstet mit Rückflusskühler, Abgasabsorption und Rührer, wird die Nitroanthrachinonverbindung, z.B. 1-Nitroanthrachinon gegebenenfalls zusammen mit einem inerten organischen Lösungsmittel, vorgelegt und auf eine Temperatur von 230° bis 280°C erhitzt, wobei sich eine dünnflüssige Schmelze bildet. Anschliessend wird unter kräftigem Rühren elementares Brom zugegeben, wobei die Dosiergeschwindigkeit zweckmässigerweise so gewählt wird, dass im Kühler ständig ein leichter Bromrückfluss aufrechterhalten bleibt. Nach einer Reaktioszeit von ca. 3 bis 10 Stunden wird kein Brom mehr verbraucht und im Dünnschichtchromatogramm ist kein Nitroanthrachinon mehr nachweisbar. Zur Aufarbeitung kühlt man das Reaktionsgemisch auf eine Temperatur von ca. 200°C ab und lässt es sodann unter kräftigem Rühren in die ein- bis zweifache Menge Wasser einlaufen, wobei sich eine Suspension bildet. Diese wird anschliessend während ca. 1 Stunde unter Rückfluss gekocht, dabei gehen Nebenprodukte in Lösung. Danach lässt man abkühlen, filtriert, wäscht den isolierten Festkörper mit heissem Wasser und trocknet das Produkt im Vakuum. Man erhält so mit einer Ausbeute von über 95% die entsprechenden Bromanthrachinone, im vorliegenden Falle 1-Bromanthrachinon mit einem Gehalt von 96 bis 97%

Die Erfindung wird durch die folgenden Beispiele erläutert;-Teile bedeuten Gewichtsteile und Prozente Gewichtsprozente.

**Beispiel 1**

In einem 1 l-Doppelmantelkolben mit Propeller, oder Ankerrührer, Bromeinleitung mit Dosierpumpe, Rückflusskühler mit Anschluss an Abgasadsorption werden 1265 Gew.-Teile 1-Nitroanthrachinon und 60 Gew.-Teile Nitrobenzol vorgelegt, das Gemisch auf 240°C erhitzt und bei dieser Temperatur mit Hilfe der Dosierpumpe unter kräftigem Rühren elementares Brom eingepumpt. Die Dosiergeschwindigkeit wird so gewählt, dass im Rückflusskühler ständig ein leichter Bromrückfluss aufrechterhalten bleibt. Nachdem etwa 200 bis 220 ml Brom innerhalb von 10 Stunden zudosiert wurden, wird kein Brom mehr vom Reaktionsgemisch aufgenommen und im Dünnschichtchromatogramm ist kein 1-Nitroanthrachinon mehr nachweisbar. Nun wird das Gemisch auf 200°C gekühlt und die Schmelze durch Untenauslauf in 2 l kräftig gerührtes Wasser abgelassen. Die Suspension wird darauf unter Rückfluss gekocht und über einen Wasserabscheider das

Nitrobenzol abgetrennt. Nach ca. 1 Stunde wird die Suspension bis auf etwa 80°C abgekühlt, filtriert, das Nutschgut mit heissem Wasser gewaschen und im Vakuum bei 120°C getrocknet. Auf diese Weise werden 1400 Gew.-Teile 1-Bromanthrachinon mit einem Gehalt von 96-97% erhalten. Ausbeute, bezogen auf 1-Nitroanthrachinon: 97,5%.

**Beispiel 2**

In einer Laborapparatur, wie in Beispiel 1 beschrieben, werden 1265 Gew.-Teile 1-Nitroanthrachinon und 30 Gew.-Teile 1,4-Nitrobrombenzol auf 275°C erhitzt und bei dieser Temperatur elementares Brom so zudosiert, dass im Kühler ständig ein sehr leichter Bromrückfluss auftritt. Nachdem etwa 190 ml Brom innerhalb von ca. 3 Stunden zudosiert wurden, wird vom Reaktionsgemisch kein Brom mehr aufgenommen und im Dünnschichtchromatogramm ist kein 1-Nitroanthrachinon mehr nachweisbar. Nun wird das Gemisch auf 200°C gekühlt und in 1,5 l gut gerührtes Nitrobenzol abgelassen. Die Suspension wird nochmals bis auf 150°C erhitzt, wobei praktisch alles 1-Bromanthrachinon in Lösung geht. Beim Abkühlen kristallisiert das Produkt in grossen tiefgelben Kristallen aus. Diese werden abfiltriert, mit wenig Nitrobenzol gewaschen und bei 120°C im Vakuum getrocknet. Dabei werden 1320 Gew.-Teile 1-Bromanthrachinon mit einem Gehalt von 99,5-100% erhalten. Die Ausbeute, bezogen auf 1-Nitroanthrachinon, beträgt 92%.

**Beispiel 3**

In einer Laborapparatur, wie in Beispiel 1 beschrieben, werden 1265 Gew.-Teile 2-Nitroanthrachinon in Anwesenheit von 60 Gew.-Teilen Nitrobenzol bei 270°C mit elementarem Brom umgesetzt. Ist die Umsetzung beendet, wird die Schmelze bei 200°C unter kräftigem Rühren in 2000 Gew.-Teile Methanol abgelassen. Die Suspension wird 1 Stunde unter Rückfluss gekocht, bis auf 50°C gekühlt, filtriert und getrocknet. Dabei werden 1390 g 2-Bromanthrachinon (Ausbeute 97%) mit einem Gehalt von 98% erhalten.

**Beispiel 4**

In einem Emailkessel, der mit Hochtemperatur-Heizanlage, Intensivkühler, Bromeinleitungsrohr und Kühler mit Anschluss an eine Abgasadsorptionsanlage ausgerüstet ist, werden 3300 Teile Nitrobenzol vorgelegt und unter Rühren 3290 Teile 1-Nitroanthrachinon als ca. 98%ige Ware eingetragen. Das Gemisch wird auf 245°C geheizt, wobei ein leichter Ueberdruck (0,5 bis 0,7 atü) entsteht, welcher zum Erreichen der benötigten Reaktionstemperatur mit Hilfe eines Druckreglers aufrechterhalten wird. Nun werden bei 245-250°C innerhalb 10 bis 12 Stunden 1800 Teile Brom in das Reaktiosgemisch dosiert. Die Dosierungsgeschwindigkeit wird so geregelt, dass im Kühler ständig ein leichter Bromrückfluss aufrechterhalten bleibt. Wird praktisch kein Brom mehr aufgenommen und ist laut Chromatogramm die Menge des 1-Nitroanthrachinons unter 1% gesunken, so ist die Reaktion beendet.

Anschliessend wird das Gemisch auf 170°C gekühlt, kurz mit Stickstoff ausgeblasen und in einen Kristallisationskessel abgelassen. Dort wird es nach Temperaturprogramm auf 20°C (bzw. auf 0°C) abgekühlt, die Kristalle werden abgenutscht und 2 bis 3 mal mit je 500 Teilen Nitrobenzol gewaschen.

Das Nutschgut wird nitrobenzolfeucht weiterverarbeiet oder getrocknet. Es werden ca. 3400 Teile 1-Bromanthrachinon zu 98-99% = ca. 3350 Teile 100%, oder 90% der Theorie, auf 1-Nitroanthrachinon berechnet, erhalten.

Die Mutterlauge, ca. 3500 kg, wird destilliert, das Nitrobenzol wieder eingesetzt und der Destillationsrückstand verbrannt.

Das Waschnitrobenzol kann ohne Reinigung wiederverwendet werden.

**Beispiel 5**

In einem Emailkessel werden 750 Teile Nitrobenzol vorgelegt und unter Rühren 750 Teile 1-Nitroanthrachinon 100% als ca. 98%ige Ware eingetragen. Das Gemisch wird auf 245°C geheizt und bei 245-250°C und leichtem Ueberdruck (0,5-0,7 atü) werden innerhalb ca. 5 Stunden 300 Teile Brom eingeleitet. Anschliessend werden 10 Stunden lang pro Stunde eine Suspension von 254 Teilen 1-Nitroanthrachion in 254 Teilen Nitrobenzol und gleichzeitig pro Stunde 150 Teile Brom flüssig in das Reaktionsgemisch gedrückt. Ist die Zugabe des 1-Nitroanthrachinons beendet, so werden zur Nachreaktion noch pro Stunde 20 Teile Brom flüssig zugedrückt bis praktisch kein Brom mehr aufgenommen wird und laut Chromatogramm (LC) die Menge des 1-Nitroanthrachinons unter 1 % gesunken ist. Darauf wird das Gemisch auf 170°C gekühlt, kurz mit Stickstoff

ausgeblasen und in einen Kristallisationskessel abgelassen. Dort wird es nach einem Temperaturprogramm auf 20°C (bzw. auf 0°C) gekühlt; die Kristalle werden abgenutscht und zwei- bis dreimal mit je 500 Teilen Nitrobenzol gewaschen. Ausbeute und Qualität entsprechen den von Beispiel 4.

**Beispiel 6**

In einem Emailkessel werden 750 Teile Nitrobenzol vorgelegt und unter Rühren 750 Teile 1-Nitroanthrachinon 100% als ca. 98%ige Ware eingetragen. Das Gemisch wird auf 220°C geheizt und weitere 1550 Teile 1-Nitroanthrachinon werden eingetragen. Das Gemisch wird nun auf 240°C geheizt und es werden bei 240-245°C innerhalb 10 Stunden 1200 Teile Brom eingeleitet. Die Dosierungsgeschwindigkeit wird so geregelt, dass im Kühler immer ein leichter Bromrückfluss aufrechterhalten bleibt. Anschliessend werden ebenfalls bei 240-245°C 7 Stunden lang pro Stunde 335 Teile 1-Nitroanthrachinon und 187 Teile Brom, insgesamt 2345 Teile, 1-Nitroanthrachinon und 1309 Teile Brom zum Reaktionsgemisch dosiert. Ist die Dosierung von 1-Nitroanthrachinon beendet, so werden zur Nachreaktion pro Stunde noch 20 Teile Brom eingeleitet, bis praktisch kein Brom mehr aufgenommen wird und laut Chromatogramm (LC) die Menge des 1-Nitroanthrachinons unter 1% gesunken ist.

Nach beendeter Reaktion wird das Reaktionsgemisch auf 200°C gekühlt, kurz mit Stickstoff ausgeblasen und von den 6043 Teilen Schmelze werden 5103 Teile unter kräftigem Rühren in eine Vorlage mit 4600 Teilen Chlorbenzol abgelassen. Das Gemisch in der Vorlage wird auf Rückfluss (130°C) erhitzt, wobei eine klare rotbraune Lösung entsteht. Beim Abkühlen kristallisiert das Produkt in gelben Nadeln aus. Diese werden bei 20°C abfiltriert, mit 1000 Teilen Chlorbenzol in mehreren Portionen gewaschen und getrocknet. Dabei wird ein ca. 98%iges 1-Bromanthrachinon mit einer Ausbeute von ca. 90% der Theorie erhalten.

Im Ansatzkessel sind etwa 940 Teile Schmelze zurückgeblieben. Dazu werden bei 245-250°C 15 Stunden lang pro Stunde 335 Teile 1-Nitroanthrachinon und 187 Teile Brom, insgesamt 5025 Teile 1-Nitroanthra-chinon und 2805 Teile Brom dosiert. Ist die Dosierung von 1-Nitro-anthrachinon beendet, so werden zur Nachreaktion pro Stunde noch 20 Teile Brom eingeleitet, bis praktisch kein Brom mehr aufgenommen wird und im Chromatogramm (LC) die Menge des 1-Nitroanthrachinons unter 1% gesunken ist. Nach beendeter Reaktion wird das Reaktionsgemisch auf 200°C gekühlt, kurz mit Stickstoff ausgeblasen und von den 6446 Teilen Schmelze werden 5506 Teile in eine Vorlage abgelassen, in der sich 5700 Teile Chlorbenzol befinden. Das Gemisch wird kurz auf Rückfluss geheizt, dann auf 20°C gekühlt, die Kristalle werden abfiltriert, mit Chlorbenzol gewaschen und getrocknet.

**Beispiel 7**

In einer Apparatur wie in Beispiel 1 beschrieben werden 1265 Teile 1-Nitroanthrachinon durch Erhitzen auf 240°C zum Schmelzen gebracht und dann bei 245-250°C unter kräftigem Rühren elementares Brom eingepumpt. Die Dosiergeschwindigkeit wird so gewählt, dass im Rückflusskühler ständig ein leichter Bromrückfluss aufrechterhalten bleibt. Nachdem etwa 624 Teile Brom innerhalb von ca. 10 Stunden zudosiert wurden, wird kein Brom mehr vom Reaktionsgemisch aufgenommen und im Dünnschichtchromatogramm ist kein 1-Nitroanthrachinon mehr nachweisbar. Nun wird das Gemisch auf 200°C gekühlt und die Schmelze in 1200 Teile kräftig gerührtes Chlorbenzol abgelassen. Die Suspension wird auf 20°C gekühlt, filtriert, das Nutschgut mit Chlorbenzol gewaschen und die Kristalle im Vakuum bei 120°C getrocknet. Auf diese Weise werden 1350 Teile 1-Bromanthrachinon mit einem Gehalt von 99,0-99,5% erhalten. Die Ausbeute, bezogen auf 1 Nitroanthrachinon, beträgt 96%.

**Beispiel 8**

Eine 2 m lange Blasensäule mit einem Durchmesser von 100 mm und 10 Siebböden wird am Kopf mit 6,5 Teilen/Stunde 1-Nitroanthrachinon beschickt, welches durch Erhitzen auf 250-260°C zum Schmelzen gebracht worden ist. In diese Schmelze werden im Gegenstrom 3 Teile/Stunde an Brom eigeleitet, das in einem Verdampfer verflüchtigt und auf 240°C erwärmt wurde. Das 1-Nitroanthrachinon wird beim Durchströmen der Säule vollständig in 1-Bromanthrachinon überführt, welches die Säule über einen Siphon verlässt und in kräftig gerührtes Wasser abgelassen wird. Die wässrige Suspension wird bei etwa 80°C filtriert, das Produkt mit heissem Wasser gewaschen und im Vakuum bei 120°C getrocknet. Auf diese Weise werden 96-97%iges 1-Bromanthrachinon mit einer Ausbeute von 97,5% der Theorie, bezogen auf das eingesetzte 1-Nitroanthrachinon, erhalten.

**Beispiel 9**

In einem Druckgefäss (für einen Ueberdruck bis 3 atü), das mit einem Druckregler ausgestattet ist, über den Abgase bei einem einstallbaren, leichten Ueberdruck entweichen können, werden 450 Teile Nitrobenzol und 300 Teile 1,5-Dinitroanthrachinon auf 260°C erhitzt, wobei ein Ueberdruck von ca. 1,2 atü entsteht. Bei Aufrechterhalten dieser Temperatur und dem angegebenen Ueberdruck werden innerhalb ca. 8 Stunden 240 Teile Brom in das Gemisch gepumpt, wobei die Abgase über den Druckregler entweichen. Wird kein Brom mehr vom Reaktionsgemisch aufgenommen und ist im Dünnschichtchromatogramm weder 1,5-Dinitroanthrachinon noch 1-Brom-5-nitroanthrachinon nachweisbar, so wird das Gemisch auf 200°C gekühlt, mit Stickstoff ausgeblasen und mit einem für die Kristallisation optimalen Temperaturprogramm auf 20°C gekühlt.

Der dicke Kristallbrei wird anschliessend auf eine Nutsche abgelassen, filtriert, mit Nitrobenzol gewaschen und getrocknet. Dabei werden 360 Teile (98% der Theorie) dünnschichtchromatographisch reines 1,5-Dibromanthrachinon erhalten.

**Patentansprüche**

1. Verfahren zur Herstellung reiner Bromanthrachinone durch Denitrobromieren entsprechender Nitroanthrachinone, dadurch gekennzeichnet, dass man auf Nitroanthrachinone der Formel I

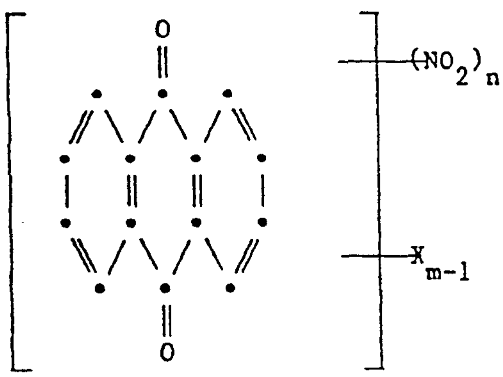

(I),

worin X für Hydroxy, Mercapto, Carboxy oder Halogen steht und m 1, 2, 3 oder 4 und n 1 oder 2 bedeutet, bei Temperaturen von 200° bis 350°C elementares Brom einwirken lässt.

2. Verfahren gemäss Anspruch 1, dadurch gekennzeichnet, dass man die Denitrobromierung in Gegenwart eines inerten organischen Lösungsmittels mit einem Siedepunkt von >150°C durchführt.

3. Verfahren gemäss Anspruch 2, dadurch gekennzeichnet, dass man 1 bis 500 Gew.% Lösungsmittel, bezogen auf Nitroanthrachinon, verwendet.

4. Verfahren gemäss Anspruch 2, dadurch gekennzeichnet, dass man als Lösungsmittel halogenierte Nitrobenzole, insbesondere Nitrobenzol, verwendet.

5. Verfahren gemäss den Ansprüchen 1 und 2, dadurch gekennzeichnet, dass man bei einer Temperatur von 230 bis 280°C arbeitet.

6. Verfahren gemäss den Ansprüchen 1 und 2, dadurch gekennzeichnet, dass man 0,5 bis 1,5 Mol, vorzugsweise 0,6 bis 1,0 Mol, elementares Brom pro Nitrogruppe verwendet.

7. Verfahren gemäss den Ansprüchen 1 und 2, dadurch gekennzeichnet, dass man 1-Nitroanthrachinon oder 1,5-Dinitroanthrachinon denitrobromiert.

8. Verfahren gemäss den Ansprüchen 1 und 2, dadurch gekennzeichnet, dass man das geschmolzene Reaktionsprodukt zur Aufarbeitung in Wasser oder ein organisches Lösungsmittel einträgt, in dem das Produkt bei 20°C keine oder nur eine geringe Löslichkeit besitzt, und die Suspension gegebenenfalls auf Siedetemperatur erhitzt.

**Claims**

1. A process for the preparation of pure bromoanthraquinones by denitrobrominating corresponding nitroanthraquinones, which process comprises treating nitroanthraquinones of the formula 1

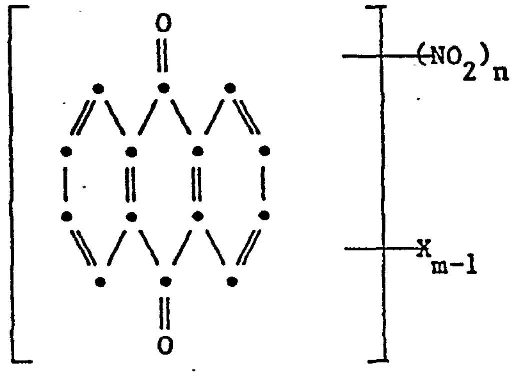

(I),

wherein X is hydroxy mercapto carboxy or halogen m is 1, 2, 3 or 4 and n is 1 or 2, with elementary bromine in the temperature range from 200° C to 350° C.

2. A process accoding to claim 1 wherein the denitrobromination is carried out in the presence of an inert organic solvent with a boiling point of >150° C.

3. A process according to claim 2, wherein 1 to 500% by weight of solvent is used, based on nitroanthraquinone.

4. A process according to claim 2, wherein the solvent employed is a halogenated nitrobenzene, in particular nitrobenzene.

5. A process according to either of claims 1 or 2, which is carried out in the temperature range from 230° to 280° C.

6. A process according to either of claims 1 or 2, wherein 0-5 to 1.5 moles, preferably 0.6 to 1 mole, of elementary bromine are used per mole of nitro group.

7. A process according to either of claims 1 or 2, wherein 1-nitroanthraquinone or 1,5-dinitroanthraquinone is denitrobrominated.

8. A process according to either of claims 1 or 2, wherein working up is effected by charging the melt of the reaction product into water or an organic solvent in which it is insoluble or only sparingly soluble at 20° C, and, if appropriate, heating the resultant suspension to boiling temperature.

**Revendications**

1. Procédé de préparation de bromoanthraquinones pures par dénitrobromation des nitroanthraquinones correspondantes, caractérisé par le fait que l'on fait agir du brome élémentaire, à des températures de 200° à 350° C, sur des nitroanthraquinones de formule I

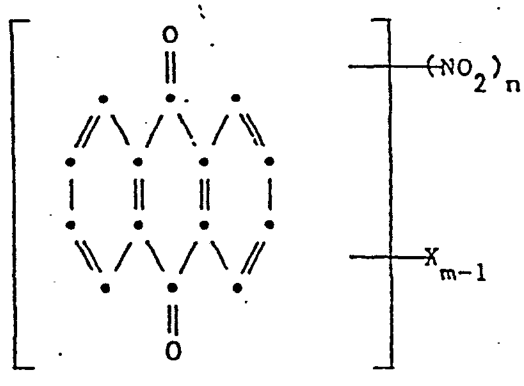

(I)

dans laquélle X représente un groupe hydroxy, mercapto, carboxy ou un halogène et m représente 1, 2, 3 ou 4 et n représente 1 ou 2.

2. Procédé selon la revendication 1, caractérisé par le fait que l'on réalise la dénitrobromation en présence d'un solvent organique inerte ayant un point d'ébullition de plus de 150° C.

3. Procédé selon la revendication 2, caractérisé par le fait que l'on utilise de 1 à 500 % en poids de solvant, par rapport à la nitroanthraquinone.

4. Procédé selon la revendication 2, caractérisé par le fait que l'on utilise comme solvant des nitrobenzènes halogénés, en particulier le nitrobenzène.

5. Procédé selon les revendications 1 et 2, caractérisé par le fait que l'on travaille à une température comprise entre 230 et 280° C.

6. Procédé selon les revendications 1 et 2, caractérisé par le fait que l'on utilise de 0,5 à 1,5 mole, de préférence de 0,6 à 1,0 mole, de brome élémentaire par groupe nitro.

7. Procédé selon les revendications 1 et 2, caractérisé, par le fait que l'on effectue la dénitrobromation de la 1-nitroanthraquinone ou de la 1,5-dinitroanthraquinone.

8. Procédé selon les revendications 1 et 2, caractérisé par le fait que, pour le traitement final, on introduit le produit réactionnel fondu dans de l'eau ou dans un solvant organique, dans lequel, à 20° C, le produit n'est pas soluble ou n'est que peu soluble, et on chauffe la suspension éventuellement à la température d'ébullition.